# EUROPÄISCHE PATENTSCHRIFT

(11) **EP 4 125 110 B1**
(45) Veröffentlichungstag und Bekanntmachung des Hinweises auf die Patenterteilung: **20.11.2024**
(21) Anmeldenummer: 21188754.2
(22) Anmeldetag: 30.07.2021
(51) Int. Cl.: H01J 35/06, H01J 35/24

(54) **RÖNTGENRÖHRE FÜR EINE STEREOSKOPISCHE BILDGEBUNG**
X-RAY TUBES FOR STEREOSCOPIC IMAGING
TUBE À RAYONS X POUR UNE IMAGERIE STÉRÉOSCOPIQUE

(43) Veröffentlichungstag der Anmeldung: 01.02.2023
(73) Patentinhaber: Siemens Healthineers AG, 91301 Forchheim (DE)
(72) Erfinder: Deuringer, Josef, 91074 Herzogenaurach (DE); Freudenberger, Jörg, 90562 Kalchreuth (DE); Fritzler, Anja, 91052 Erlangen (DE); Geithner, Peter, 91058 Erlangen (DE); Hackenschmied, Peter, 90425 Nürnberg (DE); Jud, Christoph, 90419 Nürnberg (DE)
(74) Vertreter: Siemens Healthineers Patent Attorneys

(56) Entgegenhaltungen:
- US-A1- 2010 189 221
- US-A1- 2015 043 712
- US-A1- 2015 078 523
- US-A1- 2020 352 531
- US-B2- 9 427 198

## Beschreibung

Die Erfindung betrifft eine Röntgenröhre für eine stereoskopische Bildgebung und ein Verfahren zur Generierung von Röntgenstrahlung für eine stereoskopische Bildgebung.

Die in der medizinischen Bildgebung zu untersuchenden Objekte, insbesondere die Patienten, sind dreidimensional. Daher werden bei manchen Anwendungen Aufnahmen aus unterschiedlichen Richtungen des Patienten erfasst, um Informationen über die Tiefe zu erlangen. Solche Anwendungen im Bereich der Röntgenstrahlen-basierten Bildgebung finden beispielsweise in Computertomographie-Geräten oder in C-Bögen bei einer Angiographie statt.

Die räumliche Wahrnehmungsfähigkeit des Menschen wird bei diesen Anwendungen regelmäßig nicht ausreichend genutzt. Um diese Fähigkeit zu nutzen, sind zwei Röntgenaufnahmen mit einem geringen Winkelversatz nötig, was beispielsweise bereits in der DE 30 18 541 A1 beschrieben ist. Die aus diesen Aufnahmen rekonstruierten Bilder werden idealerweise über eine Optik jeweils einem Auge eines Betrachters zugeführt. Dadurch entsteht ein dreidimensionaler Eindruck beim Betrachter. Für den Winkelversatz der Röntgenaufnahmen müssen zwei Brennflecke in einem gewissen Abstand, vorzugsweise dem Augenabstand, vorhanden sein.

Eine Möglichkeit ist die Verwendung von zwei separaten herkömmlichen Drehanoden (siehe z.B. US 8,180,017 B2, DE 10 2011 081 550 A1 sowie Fig. 4 der US 9,251,992 B), was allerdings aufgrund der Baugröße schwierig umzusetzen sein kann. Stehanoden-Röntgenröhren gemäß der Fig. 2 der US 9,251,992 B2 benötigen zwar weniger Bauraum, liefern allerdings regelmäßig zu wenig Leistung. Eine mechanische Bewegung einer einzigen herkömmlichen Röntgenröhre ist üblicherweise zu langsam.

Zwei Brennflecke auf einer einzigen Anode insbesondere durch Ablenkung des Elektronenstrahls unterzubringen, ist eine weitere aus dem Stand der Technik bekannte Alternative (WO 2012 / 123 843 A1, DE 198 10 346 C1, US 9,554,757 B2). Dabei liegen beispielsweise zwei Brennflecke auf dem Anodenrand einer Drehanode, wobei in diesem Fall bis zu der Hälfte des Detektors im Anodenschatten liegen kann. Aus der DE 198 10 346 C1 ist bekannt, dass eine Drehung zumindest einer der beiden Brennflecke nötig sein kann. Die Situation kann durch eine relativ große, aber dadurch teure Anode verbessert werden. Alternativ kann die Anode gekippt werden, wodurch in einer herkömmlichen Röntgenröhre der Röntgenstrahlung-abschwächende Fokuskopf im Strahlengang liegt. Die EP 3 751 593 A1 offenbart diesbezüglich die Verwendung einer Kathode mit einem Röntgenstrahlen-transparenten Material. Aus der US 9,554,757 B2 ist die Verwendung eines Feldeffekt-Emitters mit Nanoröhrchen auf Kohlenstoff-Basis bekannt.

DE 36 35 948 A1 beschreibt eine Strahlenblende für ein Stereobild-Röntgendiagnostikgerät. DE 35 32 822 A1 offenbart eine Stereoröntgenröhre, bei welcher der Abstand der Brennflecke fest ist. DE 10 2007 027 451 A1 offenbart die Verwendung eines Röntgenstrahlteilers mittels röntgenoptischer Elemente, wobei die bekannten Röntgenoptiken regelmäßig nur relativ kleine Ablenkwinkel ermöglichen, so dass die darin beschriebene Anordnung üblicherweise technisch nur mit großen Abständen zwischen Optiken und Detektor möglich ist.

Den zuvor beschriebenen Ausführungen ist gemein, dass der nutzbare Aufenthaltsbereich der Brennflecke relativ klein ist. Dadurch kann der Abstand der Brennflecke typischerweise nur in einem sehr engen Bereich angepasst werden (weniger als +/- 5 mm). Der Augenabstand beträgt beim Menschen durchschnittlich 65 mm (Männer) bzw. 62 mm (Frauen), kann aber je nach Konstitution und Größe im Bereich zwischen ca. 52 mm und 78 mm variieren. Die bisher diesseits bekannten Ausführungen sind nicht in der Lage, den Abstand der Brennflecke so einzustellen, dass ein für den individuellen Betrachter optimiertes Bild entsteht. Dadurch kann unter Umständen die räumliche Wahrnehmungsfähigkeit trotz derartiger Röntgenaufnahmen nicht genutzt werden.

US 2015/043 712 A1 beschäftigt sich generell mit der Radiographie und insbesondere mit Geräten und Verfahren für das Erzeugen von stereoskopischen Bildern mittels eines Radiographiesystems. US 2015/0 078 523 A1 betrifft mindestens eine Röntgenröhrenanordnung, wobei jede Röntgenröhrenanordnung eine Vielzahl an Kathoden und wenigstens eine Anode aufweist. In US 9,427,198 B2 ist ein System und ein Verfahren für live 3D-Röntgenbildgebung offenbart.

Der Erfindung liegt die Aufgabe zu Grunde, eine Röntgenröhre für eine stereoskopische Bildgebung und ein Verfahren zur Generierung von Röntgenstrahlung für eine stereoskopische Bildgebung, bei welchen der Abstand zwischen den Brennflecken in einem größeren Bereich einstellbar ist.

Die Aufgabe wird durch die Merkmale der unabhängigen Ansprüche gelöst. Vorteilhafte Ausgestaltungen sind in den Unteransprüchen beschrieben.

Die erfindungsgemäße Röntgenröhre für eine stereoskopische Bildgebung weist
- ein evakuiertes Röntgenröhrengehäuse,
- eine in dem Röntgenröhrengehäuse angeordnete Elektronenemittervorrichtung,
- eine in dem Röntgenröhrengehäuse angeordnete Anodeneinheit zur Generierung von Röntgenstrahlung für die stereoskopische Bildgebung in Abhängigkeit von auf zwei Brennflecken eintreffenden Elektronen und
- eine Steuereinheit auf,
- wobei die Elektronenemittervorrichtung einen ersten Feldeffekt-Emitter mit einer ersten Emitterfläche und einen zweiten Feldeffekt-Emitter mit einer zweiten Emitterfläche umfasst,
- wobei die erste Emitterfläche und die zweite Emitterfläche ein im Wesentlichen Röntgenstrahlen-transparentes Material aufweisen,
- wobei die erste Emitterfläche und/oder die zweite Emitterfläche derart segmentiert ist, dass ein Anteil der Elektronen-emittierenden Fläche relativ zur jeweiligen gesamten Emitterfläche durch selektives Schalten der Emittersegmente einstellbar ist,
   dadurch gekennzeichnet,
- dass die erste Emitterfläche und die zweite Emitterfläche im Strahlengang der in dem jeweiligen Brennfleck generierten Röntgenstrahlung angeordnet sind sowie
- dass die Steuereinheit zum Einstellen eines Abstands zwischen den beiden Brennflecken durch ein Vorgeben des Anteils der Elektronen-emittierenden Fläche der ersten Emitterfläche und/oder der zweiten Emitterfläche ausgebildet ist.

Vorteilhafterweise kann die Elektronen-emittierende Fläche beliebig konfiguriert werden, so dass der Abstand zwischen den beiden Brennflecken über einen größeren Bereich einstellbar ist. Dies wird insbesondere dadurch ermöglicht, dass aufgrund der Segmentierung der Emitterflächen der Anteil der Elektronen-emittierenden Fläche flexibel einstellbar ist. Das bedeutet insbesondere, dass einzelne Segmente der ersten Emitterfläche und/oder der zweiten Emitterfläche angeschaltet oder abgeschaltet werden können, wodurch die Position der jeweiligen Elektronenströme variiert werden kann. Letztlich kann allein durch das Variieren der Position der Elektronen-emittierenden Fläche der ersten Emitterfläche oder der zweiten Emitterfläche der Abstand zwischen den beiden Brennflecken beeinflusst werden. Besonders vorteilhafterweise kann der Abstand über einen noch größeren Bereich beeinflusst werden, wenn beide Emitterflächen segmentiert sind.

Die Verwendung der Feldeffekt-Emitter ist insbesondere vorteilhaft, weil aufgrund deren hohen Elektronenstromdichte Fokussierungselemente und/oder Ablenkeinheiten regelmäßig verzichtbar sind. Außerdem weisen die Emitterflächen solcher Feldeffekt-Emitter üblicherweise das Röntgenstrahlen-transparente Material auf, so dass die Röntgenstrahlung beim Durchdringen der ersten Emitterfläche und der zweiten Emitterfläche vergleichsweise wenig abgeschwächt wird.

Die Röntgenröhre ist insbesondere also für solche Röntgenstrahlen-basierte Anwendungen der stereoskopischen Bildgebung vorteilhaft, bei welchen die Röntgenstrahlen an verschiedenen Brennflecken konsekutiv erzeugt und somit mindestens zwei Aufnahmen aus unterschiedlichen Einfallwinkeln am Röntgendetektor erfasst werden, wobei die beiden Aufnahmen dann einem Nutzer angezeigt werden können, bei welchem dabei der räumliche Eindruck entsteht. Diese Anwendungen können insbesondere eine Angiographie oder Computertomographie betreffen.

Die Anodeneinheit umfasst mindestens eine Anode, kann je nach Ausführungsform lediglich eine einzige Anode umfassen. Die Anode ist typischerweise drehbar gelagert, so dass durch die bei der Drehung stattfindende Kühlung eine höhere Hubtemperatur ermöglicht wird und somit ein höherer Elektronenstrom auf der Anode als bei einer Stehanode auftreffen kann. Auf der Rückseite umfasst die Anode beispielsweise Grafit zur Entwärmung der Anodenoberfläche, welche im Bereich der Brennflecke beispielsweise Wolfram und/oder Molybdän aufweist.

Die Elektronen werden von der Elektronenemittervorrichtung Richtung der Anodeneinheit beschleunigt und generieren bei der Wechselwirkung in den beiden Brennflecken typischerweise konsekutiv die Röntgenstrahlung. Grundsätzlich kann die Röntgenröhre auch dazu ausgebildet sein, in beiden Brennflecken gleichzeitig Röntgenstrahlung generieren zu können. Die Brennflecke können Teil zumindest einer Brennbahn sein. Die generierte Röntgenstrahlung weist üblicherweise eine maximale Energie von bis zu 150 keV in Abhängigkeit von der zwischen der Elektronenemittervorrichtung und der Anodeneinheit angelegten Beschleunigungsspannung auf.

Eine äußere Form des ersten Brennflecks und/oder des zweiten Brennflecks kann grundsätzlich rund sein, ist regelmäßig allerdings nicht rund. Typischerweise ist die äußere Form viereckig, beispielsweise rechteckig oder rautenförmig. Typischerweise ist die äußere Form in radialer Richtung der Anode größer als in Phi-Richtung, d. h. in Umfangsrichtung der Anode.

Konsekutiv (oder alternierend) bedeutet, dass die generierte Röntgenstrahlung zu einem Auslesezeitpunkt jeweils einem Brennfleck zugeordnet werden kann. Eine Frequenz der konsekutiven Röntgenstrahlerzeugung kann auf eine Auslesefrequenz des Röntgendetektors abgestimmt sein, welcher typischerweise über einen gewissen Zeitraum die eintreffenden Photonen integriert, bevor eine Aufnahme zu einem bestimmten Auslesezeitpunkt erzeugt wird. Nach einer ersten Aufnahme zu einem ersten Auslesezeitpunkt mit der Röntgenstrahlung des ersten Brennflecks wird regelmäßig eine zweite Aufnahme zu einem zweiten Auslesezeitpunkt mit der Röntgenstrahlung des zweiten Brennflecks erfasst. Dieses konsekutive Generieren kann beliebig wiederholt werden, beispielsweise während der gesamten Dauer der bildgebenden Untersuchung erfolgen.

Das Röntgenröhrengehäuse kann Glas und/oder Metall umfassen. Falls die erste Emitterfläche und/oder die zweite Emitterfläche keinen Teil des Röntgenröhrengehäuses bilden, weist das Röntgenröhrengehäuse ein Röntgenstrahlenaustrittfenster insbesondere dort auf, wo der Strahlengang der in dem jeweiligen Brennfleck generierten Röntgenstrahlung aus dem Röntgenröhrengehäuse austreten soll. Das Röntgenröhrengehäuse ist typischerweise von einem Kühlmedium umgegeben.

Der erste Feldeffekt-Emitter und der zweite Feldeffekt-Emitter sind regelmäßig derart angeordnet, dass ein gewisser Abstand zwischen der ersten Emitterfläche und der zweiten Emitterfläche besteht. Der erste Feldeffekt-Emitter und der zweite Feldeffekt-Emitter können grundsätzlich derart angeordnet sein, dass die erste Emitterfläche und die zweite Emitterfläche eine im Wesentlichen zusammenhängende Emitterfläche bilden. In diesem Fall ist also ein Abstand zwischen den beiden Emitterflächen vorzugsweise minimal. Dabei sind der erste Feldeffekt-Emitter und der zweite Feldeffekt-Emitter physisch dadurch zu unterscheiden, dass jeder eine separate Plattform, beispielsweise in Form eines nicht-zusammenhängenden Substrats, aufweist.

Alternativ können der erste Feldeffekt-Emitter und der zweite Feldeffekt-Emitter physisch nicht unterscheidbar sein, sondern derart integriert auf einer einzigen Plattform ausgebildet sein, dass die Elektronenemittervorrichtung eine zusammenhängende Emitterfläche aufweist. In diesem Fall kann die Unterscheidung zwischen dem ersten Feldeffekt-Emitter und dem zweiten Feldeffekt-Emitter dadurch erfolgen, dass softwareseitig oder elektronisch diese zusammenhängende Emitterfläche in zwei nebeneinander angeordnete Teilbereiche unterteilt ist, wobei der erste Teilbereich die erste Emitterfläche und der zweite Teilbereich die zweite Emitterfläche bildet. In diesem Fall ist es denkbar, dass die erste Emitterfläche und die zweite Emitterfläche innerhalb der zusammenhängenden Emitterfläche neu verteilt werden kann.

Es ist vorteilhaft, wenn die erste Emitterfläche und die zweite Emitterfläche im Sinne eines maximal emittierbaren Elektronenstroms vergleichbar sind. Die Segmentierung der beiden Emitterflächen kann homogen sein, sprich sie können in im Wesentlichen gleich leistungsfähige mehrere Elektronenstrom-Emissionsbereiche unterteilt sein, oder heterogen sein. Die Segmentierung kann rasterförmig oder kreissegmentförmig sein. Die rasterförmige Segmentierung kann einer pixelartigen Segmentierung entsprechen mit vorzugsweise gleich großen Pixeln. Es ist besonders vorteilhaft, wenn eine elektrische Kontaktierung der beiden Emissionsflächen durch den jeweiligen Feldeffekt-Emitter vorzugsweise derart ausgestaltet ist, dass Röntgenstrahlen-intransparente Materialien sich weitestgehend außerhalb des Strahlengangs befinden.

Die gesamte Emitterfläche wird jeweils durch die erste Emitterfläche oder die zweite Emitterfläche vorgegeben. Die zusammenhängende Emitterfläche entspricht der gesamten Emitterfläche der ersten Emitterfläche plus der gesamten Emitterfläche der zweiten Emitterfläche. Die Elektronen-emittierende Fläche ist als diejenige Fläche definiert, aus welcher Elektronen die jeweilige Emitterfläche verlassen und insbesondere ohne Abschwächung durch ein Sperrgitter auf der Anodeneinheit auftreffen können. Der Anteil der Elektronen-emittierenden Fläche relativ zur jeweiligen gesamten Emitterfläche kann zwischen 0%, insbesondere wenn in diesem Moment in dem jeweils anderen Brennfleck Röntgenstrahlung generiert wird, und 100%, insbesondere wenn in einem anderen Moment in dem zugeordneten Brennfleck die Röntgenstrahlung generiert wird, liegen. Typischerweise wird der Anteil bei Röntgenstrahlerzeugung kleiner 100% sein, beispielsweise in einem Bereich zwischen 0,5% und 50% sein, insbesondere 2% betragen, so dass die Elektronen-emittierende Fläche innerhalb der jeweiligen gesamten Emitterfläche bei gleichbleibender Intensität des Elektronenstroms verschoben werden kann.

Die Elektronenemission bei Feldeffekt-Emitter wird typischerweise durch das Anlegen einer Gatespannung erwirkt, welche durch das in den Spitzen der Nanoröhrchen auftretenden elektrischen Felds die Elektronen aus diesen Nanoröhren extrahiert, wodurch der Elektronenstrom gebildet wird. Zusätzlich zum Schalten mittels der Gatespannung kann das Sperren eines generierten Elektronenstroms mittels eines Sperrgitters erfolgen. Das selektive Schalten der Emittersegmente umfasst zumindest das Anlegen der Gatespannung und optional das Anlegen einer Sperrspannung am Sperrgitter. Das selektive Schalten umfasst also insbesondere das Anschalten und das Abschalten, beispielsweise der angelegten Gatespannung und/oder der den Nanoröhrchen vorgeschalteten Strombegrenzungseinheiten. Selektiv schaltbar bedeutet insbesondere, dass vorzugsweise jedes Emittersegment separat anschaltbar oder abschaltbar ist. Jedes Emittersegment weist typischerweise eine Vielzahl an Nanoröhrchen auf. Die Struktur, an welchem typischerweise die Gatespannung anliegt, weist vorzugweise ebenfalls ein Röntgenstrahlen-transparentes Material, insbesondere ein Röntgenstrahlen-transparentes Metall, auf, beispielsweise Aluminium. Das Röntgenstrahlen-transparente Material kann eine leitfähige Siliziumstruktur, insbesondere amorphes Silizium sein.

Die Röntgenröhre und/oder die Elektronenemittervorrichtung kann einen Logikbaustein umfassen, in welchem die Steuereinheit und/oder eine weitere Steuereinheit und/oder eine Schnittstelle integriert sind. Dieser Logikbaustein dient insbesondere einer Steuerung der Elektronenemission der Elektronenemittervorrichtung mittels der ersten Emitterfläche und der zweiten Emitterfläche. Der Logikbaustein, insbesondere die Steuereinheit, kann dazu ausgebildet sein, den Anteil der Elektronen-emittierenden Fläche relativ zur jeweiligen gesamten Emitterfläche einzustellen, beispielsweise indem der Logikbaustein die Emittersegmente selektiv schaltet. Der Logikbaustein kann ein FPGA-Baustein oder ein Prozessor sein.

Da die Röntgenstrahlung üblicherweise konsekutiv in den beiden Brennflecken erzeugt wird, bezieht sich der Abstand beispielsweise auf den einen, soeben für die Röntgenstrahlungserzeugung genutzten Brennfleck, sowie auf den anderen, vorher genutzten oder nachher zu nutzenden Brennfleck. Der Abstand kann beispielsweise durch eine äußere Begrenzungslinie oder einem Intensitätsschwerpunkt oder einem geometrischen Mittelpunkt der beiden Brennflecke definiert sein. In Bezug auf den medizinisch definierten Augenabstand als Distanz der beiden Pupillen, beziehen sich die in der vorliegenden Schrift angegebenen Abstände auf den jeweiligen geometrischen Mittelpunkt der beiden Brennflecke.

Das Einstellen des Abstands bedeutet insbesondere ein Verändern und/oder individuelles Regeln des Abstands. Das Einstellen des Abstands zwischen den beiden Brennflecken kann mittels der Steuereinheit dadurch erfolgen, dass der Anteil der Elektronen-emittierenden Fläche einer einzigen Emitterfläche oder beider Emitterflächen eingestellt wird. Das Vorgeben des Anteils der Elektronen-emittierenden Fläche der ersten Emitterfläche und/oder der zweiten Emitterfläche kann ein Bereitstellen eines Steuersignals und/oder das selektive Schalten, also das Abschalten und/oder Anschalten der Emittersegmente, umfassen.

Der Abstand kann beispielsweise dadurch verringert werden, dass ein zentrales Emittersegment zugeschaltet wird, welches in einem zentralen Bereich zwischen den beiden Brennflecken liegt, und/oder dass ein dezentrales Emittersegment abgeschaltet wird, welches außerhalb des zentralen Bereichs zwischen den beiden Brennflecken liegt. Das Zuschalten des zentralen Emittersegments verändert üblicherweise nicht eine maximale Ausdehnung der Elektronen-emittierenden Fläche, gemessen über beide Brennflecke. Das Abschalten des dezentralen Emittersegments verringert typischerweise die maximale Ausdehnung der Elektronen-emittierenden Fläche, gemessen über beide Brennflecke.

Dass die erste Emitterfläche und die zweite Emitterfläche im Strahlengang der in dem jeweiligen Brennfleck generierten Röntgenstrahlung angeordnet sind, bedeutet insbesondere, dass der Nutzanteil an Röntgenstrahlung zumindest teilweise die jeweilige Emitterfläche durchdringt bevor diese auf dem Röntgendetektor detektierbar ist. Die erste Emitterfläche und die zweite Emitterfläche schwächen die an dem jeweiligen Brennfleck generierte Röntgenstrahlung, insbesondere deren Nutzanteil, wenigstens minimal ab, weil physikalisch betrachtet das Röntgenstrahlen-transparente Material nicht zu 100% transparent ist. Der Strahlengang umfasst also insbesondere diejenigen Röntgenphotonen, welche unter der Annahme, dass keine Streuung im durchleuchteten Objekt stattfindet, in einer gerade Linie vom Brennfleck in Richtung des Röntgendetektors propagieren. Diejenigen Röntgenphotonen bzw. der Nutzanteil an Röntgenstrahlung werden regelmäßig Fokalstrahlung genannt. In anderen Worten wird die erste Emitterfläche und die zweite Emitterfläche nicht ausschließlich von sogenannter Extrafokalstrahlung durchdrungen. Die Extrafokalstrahlung wird beispielsweise durch die Ausgestaltung des Röntgenstrahlenaustrittfensters und/oder durch einen Kollimator und/oder durch ein Streustrahlenraster vor dem Röntgendetektor abgeschirmt. Die erste Emitterfläche und die zweite Emitterfläche sind also jeweils das erste Objekt im Strahlengang, in welchem sogenannte Streustrahlung entstehen kann.

Eine Ausführungsform sieht vor, dass das Röntgenstrahlen-transparente Material Silizium ist. Silizium-Feldeffekt-Emitter sind besonders vorteilhaft, weil diese aufgrund einer gut zu integrierenden Strombegrenzungseinheit besonders widerstandsfähig und somit langlebig sind.

Eine Ausführungsform sieht vor, dass die erste Emitterfläche und/oder die zweite Emitterfläche ein vakuumdichtes Gehäuseteil des Röntgenröhrengehäuses bilden. Diese Ausführungsform kann insbesondere bedeuten, dass das Röntgenröhrengehäuse im Bereich des Strahlengangs der in dem jeweiligen Brennfleck generierten Röntgenstrahlung durchbrochen ist und die in so einer Aussparung eingesetzten Emitterflächen das Vakuum des Röntgenröhrengehäuse aufrechterhalten können. Das durchbrochene Röntgenröhrengehäuse sowie die erste Emitterfläche und/oder die zweite Emitterfläche können also vorteilhafterweise das Vakuum innerhalb des Röntgenröhrengehäuse erhalten. Daraus folgt eine vorteilhafte geringere Bauform und aufgrund der Materialersparnis ein vorzugsweise geringeres Gewicht, als wenn das Röntgenstrahlergehäuse zusätzlich zumindest ein herkömmliches Röntgenstrahlenaustrittfenster aufweist.

Eine Ausführungsform sieht vor, dass ein Fokuskopf des ersten Feldeffekt-Emitters und/oder des zweiten Feldeffekt-Emitters Aluminium aufweist. Wenn die Röntgenröhre den Fokuskopf aufweist, kann dieser insbesondere aus Aluminium bestehen. Aluminium ist ein Röntgenstrahlen-transparentes Metall und somit besonders für den Einsatz im Fokuskopf, welcher sich als Teil der Elektronenemittervorrichtung zumindest teilweise im Strahlengang der Röntgenstrahlung befinden kann, geeignet. Der Fokuskopf ist vorteilhafterweise vergleichsweise dünn ausgeführt und/oder eher parallel zur Röntgenstrahlung ausgebildet.

Eine Ausführungsform sieht vor, dass die Röntgenröhre ferner eine Schnittstelle zum Empfang eines Abstandsignals, welches mit dem Abstand zwischen den Brennflecken korreliert, aufweist. Die Schnittstelle kann insbesondere Teil der Steuereinheit oder des Logikbausteins sein. Die Schnittstelle ermöglicht vorteilhafterweise das Vorgeben des Abstandssignals von extern an die Röntgenröhre, so dass der Abstand zwischen den beiden Brennfleck individuell angepasst werden kann.

Eine Ausführungsform sieht vor, dass die Röntgenröhre ferner eine weitere Schnittstelle zum Empfang eines Blickbewegungssignals und eine weitere Steuereinheit zum derartigen nicht-achsensymmetrischen Einstellen des Anteils der Elektronen-emittierenden Fläche in Abhängigkeit des Blickbewegungssignals aufweist, dass zumindest einer der beiden Brennflecke relativ zur rasterförmigen Segmentierung verdreht ist. Die weitere Steuereinheit kann ebenso wie die Steuereinheit als Teil des Logikbausteins ausgebildet sein. Die Steuereinheit und die weitere Steuereinheit können insbesondere softwaretechnisch abgebildet und/oder unterscheidbar sein. Das Blickbewegungssignals kann insbesondere mit einer Beobachtungsachse des Betrachters korrelieren oder diese angeben.

Eine Ausführungsform sieht vor, dass die Anodeneinheit eine einzige drehbar gelagerte Scheibenanode aufweist, deren Oberfläche im Bereich der Brennflecke im Wesentlichen einen Tellerwinkel von 0° aufweist, und dass die Scheibenanode somit zumindest abschnittsweise flach ist. Einzig heißt, dass beide Brennflecke auf derselben Scheibenanode angeordnet sind. Dass die Scheibenanode flach ist, bedeutet insbesondere, dass relativ zu einem Drehachsenabschnitt zwischen zwei Kreisen mit unterschiedlichen Radien der Tellerwinkel 0° ist und in dem Bereich zwischen den beiden Kreisen die Brennflecke liegen. In einem besonderen Beispiel ist die Scheibenanode komplett flach, wobei der eine Radius dem Radius des Umfangs der Scheibenanode entspricht und der zweite Radius gleich null ist. Der Vorteil einer solchen flachen Scheibenanode ist insbesondere, dass einer der beiden Brennflecke oder beide Brennflecke beliebig innerhalb der flachen Abschnitte der Scheibenanode verschoben werden können.

Eine in Bezug auf die vorherige Ausführungsform bevorzugte Ausführungsform sieht vor, dass die Anodeneinheit relativ zur ersten Emitterfläche und zur zweiten Emitterfläche derart gekippt angeordnet ist, dass sich eine erste Ebene, welche die erste Emitterfläche und die zweite Emitterfläche umfasst, sowie eine zweite Ebene, welche die flachen Abschnitte mit den Brennflecken umfasst, sich geometrisch schneiden. In anderen Worten sind die erste Ebene und die zweite Ebene nicht parallel. Die Definition der Ebenen dient insbesondere der Veranschaulichung der geometrischen Anordnung zueinander. Diese Ausführungsform ist insbesondere vorteilhaft, weil dadurch auf der Scheibenanode mehr elektrische Leistung in Form eines höheren Elektronenstroms deponiert werden kann.

Eine alternative Ausführungsform sieht vor, dass die Anodeneinheit eine einzige drehbar gelagerte Walzenanode mit einer zylindrischen Mantelfläche aufweist und dass die Brennflecke entlang der Mantelfläche axial versetzt angeordnet sind. Einzig heißt, dass beide Brennflecke auf derselben Walzenanode angeordnet sind. Die Walzenanode bietet den Vorteil, dass eine höhere Leistung in Form eines höheren Elektronenstroms eingesetzt werden kann. Diese Ausführungsform ist insbesondere vorteilhaft, wenn zusätzlich die weitere Steuereinheit zum nicht-achsensymmetrischen Einstellen des Anteils der Elektronen-emittierenden Fläche in Abhängigkeit des Blickbewegungssignals vorgesehen ist.

Eine zu den vorherigen Ausführungsformen alternative Ausführungsform mit zwei Anoden sieht vor, dass die Anodeneinheit eine drehbar gelagerte Telleranode, deren Oberfläche im Bereich eines Brennflecks einen Tellerwinkel größer 0° aufweist, sowie eine weitere Anode aufweist, wobei die beiden Brennflecke auf die Telleranode und die weitere Anode verteilt sind. Bei dieser Ausführungsform ist üblicherweise die Position des einen Brennflecks fest und insbesondere die Position des anderen Brennflecks veränderbar. Die Verwendung der Telleranode mit dem Tellerwinkel größer 0° ermöglicht vorzugsweise eine relativ hohe Elektronenstrahlleistung insbesondere auch für Anwendungen, die nicht Teil der beanspruchten Erfindung sind und bei denen keine stereoskopische Bildgebung erforderlich ist, beispielsweise für Kontrollscans zur Dokumentation von medizinischen Eingriffen. Diese Ausführungsform kann vorteilhafterweise einen noch größeren Abstand zwischen den beiden Brennflecken ermöglichen.

Eine zur vorherigen Ausführungsform bevorzugte Ausführungsform sieht vor, dass die Telleranode derart ausgerichtet ist, dass deren Rotationsachse parallel zu einer Ebene ist, welche die erste Emitterfläche und die zweite Emitterfläche umfasst. Diese Ausführungsform ermöglicht eine besonders kompakte Bauform einer Röntgenröhre mit zwei Anoden insofern, weil ein Abstand zwischen den beiden Anoden verringert werden kann.

Eine Ausführungsform als Weiterbildung einer der beiden vorherigen Ausführungsformen sieht vor, dass die weitere Anode eine Stehanode oder eine drehbar gelagerte Scheibenanode ist, deren Oberfläche im Bereich des einen Brennflecks im Wesentlichen einen Tellerwinkel von aufweist, und somit zumindest abschnittsweise flach ist. Wenn die Stehanode oder die Scheibenanode flach ist, kann der dieser Anode zugeordnete Brennfleck über einen größeren Bereich verschoben und somit entsprechend der Abstand flexibler eingestellt werden.

Das erfindungsgemäße Verfahren zur Generierung von Röntgenstrahlung für eine stereoskopische Bildgebung mittels einer Röntgenröhre umfasst die folgende Schritte:
- Bereitstellen eines Augenabstands eines Nutzers der Röntgenröhre in Form des Abstandsignals,
- Übermitteln des Abstandsignals an die Schnittstelle der Röntgenröhre,
- Einstellen des Abstands zwischen den beiden Brennflecken und
- Konsekutives generieren der Röntgenstrahlung für die stereoskopische Bildgebung in den beiden beabstandeten Brennflecken.

Der Nutzer der Röntgenröhre ist insbesondere ein Arzt oder eine andere entsprechend geschulte Person, welche die Röntgenröhre während einer medizinischen Anwendung verwendet, um beispielsweise einen Patienten mittels der Röntgenstrahlung für die stereoskopische Bildgebung zu untersuchen. Das Bereitstellen kann ein Abrufen eines Werts, der den Augenabstand beschreibt, aus einer Speichereinheit oder das Erfassen oder ein manuelles Eingeben des Augenabstands umfassen. Das Bereitstellen des Augenabstands kann insbesondere eine Blickbewegungsverfolgung, auch "eye-tracking" genannt, umfassen. Zusätzlich zum Augenabstand kann die Blickbewegung in Form des Blickbewegungssignals bereitgestellt werden. Das Übermitteln des Abstandssignals an die Schnittstelle der Röntgenröhre erfolgt typischerweise mittels einer Sendeeinheit, welche mit der Schnittstelle über eine drahtlose oder drahtgebundene Empfangseinheit verbunden wird. Daraufhin wird der Anteil der Elektronen-emittierenden Fläche relativ zur jeweiligen gesamten Emitterfläche durch selektives Schalten der Emittersegmente eingestellt, wodurch der Abstand zwischen den beiden Brennflecken eingestellt wird. Dieser Abstand kann insbesondere wiederholt eingestellt und/oder mehrfach verändert werden. Nach dem Einstellen der Elektronen-emittierenden Fläche wird die Röntgenstrahlung für die stereoskopische Bildgebung in den beiden beabstandeten Brennflecken konsekutiv generiert.

Eine Ausführungsform sieht vor, dass der Augenabstand mittels einer Kamera ermittelt wird. Diese Ausführungsform ist insbesondere vorteilhaft, weil die Kamera ein hochfrequentes und somit schnelles Erfassen des Augenabstands ermöglicht.

Das Computerprogrammprodukt kann ein Computerprogramm sein oder ein Computerprogramm umfassen. Das Computerprogrammprodukt weist insbesondere die Programmcodemittel auf, welche die erfindungsgemäßen Verfahrensschritte abbilden. Dadurch kann das erfindungsgemäße Verfahren definiert und wiederholbar ausgeführt sowie eine Kontrolle über eine Weitergabe des erfindungsgemäßen Verfahrens ausgeübt werden. Das Computerprogrammprodukt ist vorzugsweise derart konfiguriert, dass die Recheneinheit mittels des Computerprogrammprodukts die erfindungsgemäßen Verfahrensschritte ausführen kann. Die Programmcodemittel können insbesondere in einen Speicher der Recheneinheit geladen und typischerweise mittels eines Prozessors der Recheneinheit mit Zugriff auf den Speicher ausgeführt werden. Wenn das Computerprogrammprodukt, insbesondere die Programmcodemittel, in der Recheneinheit ausgeführt wird, können typischerweise alle erfindungsgemäßen Ausführungsformen des beschriebenen Verfahrens durchgeführt werden. Das Computerprogrammprodukt ist beispielsweise auf einem physischen, computerlesbaren Medium gespeichert und/oder digital als Datenpaket in einem Computernetzwerk hinterlegt. Das Computerprogrammprodukt kann das physische, computerlesbare Medium und/oder das Datenpaket in dem Computernetzwerk darstellen. So kann die Erfindung auch von dem physischen, computerlesbaren Medium und/oder dem Datenpaket in dem Computernetzwerk ausgehen. Das physische, computerlesbare Medium ist üblicherweise unmittelbar mit der Recheneinheit verbindbar, beispielsweise indem das physische, computerlesbare Medium in ein DVD-Laufwerk eingelegt oder in einen USB-Port gesteckt wird, wodurch die Recheneinheit auf das physische, computerlesbare Medium insbesondere lesend zugreifen kann. Das Datenpaket kann vorzugsweise aus dem Computernetzwerk abgerufen werden. Das Computernetzwerk kann die Recheneinheit aufweisen oder mittels einer Wide-Area-Network- (WAN) bzw. einer (Wireless-)Local-Area-Network-Verbindung (WLAN oder LAN) mit der Recheneinheit mittelbar verbunden sein. Beispielsweise kann das Computerprogrammprodukt digital auf einem Cloud-Server an einem Speicherort des Computernetzwerks hinterlegt sein, mittels des WAN über das Internet und/oder mittels des WLAN bzw. LAN auf die Recheneinheit insbesondere durch das Aufrufen eines Downloadlinks, welcher zu dem Speicherort des Computerprogrammprodukts verweist, übertragen werden.

Im Folgenden wird die Erfindung anhand der in den Figuren dargestellten Ausführungsbeispiele näher beschrieben und erläutert. Grundsätzlich werden in der folgenden Figurenbeschreibung im Wesentlichen gleich bleibende Strukturen und Einheiten mit demselben Bezugszeichen wie beim erstmaligen Auftreten der jeweiligen Struktur oder Einheit benannt.

Es zeigen:
Fig. 1 bis 4 eine erste Variante einer Röntgenröhre in verschiedenen Ansichten und Weiterbildungen,
Fig. 5 bis 6 eine zweite Variante der Röntgenröhre in verschiedenen Ansichten,
Fig. 7 bis 8 eine dritte Variante der Röntgenröhre in verschiedenen Ansichten,
Fig. 9 eine Elektronenemittervorrichtung und
Fig. 10 ein Verfahren zur Generierung von Röntgenstrahlung für eine stereoskopische Bildgebung mittels einer Röntgenröhre.

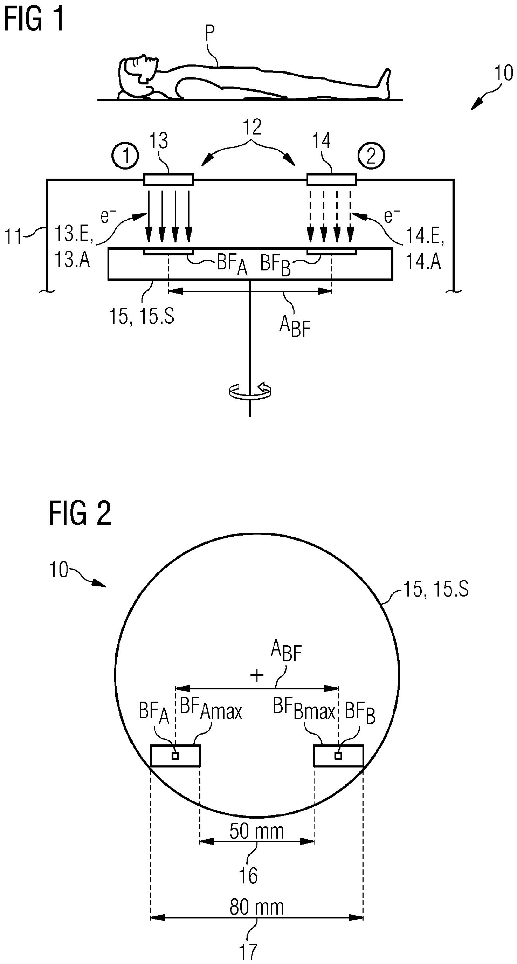

**Fig. 1** zeigt eine Röntgenröhre 10 für eine stereoskopische Bildgebung in einer Detailansicht. Die Röntgenstrahlung wird für die Untersuchung eines Patienten P durch einen nicht gezeigten Nutzer N verwendet.

Die Röntgenröhre 10 weist ein evakuiertes Röntgenröhrengehäuse 11, eine in dem Röntgenröhrengehäuse 11 angeordnete Elektronenemittervorrichtung 12 und eine in dem Röntgenröhrengehäuse 11 angeordnete Anodeneinheit 15 zur Generierung von Röntgenstrahlung für die stereoskopische Bildgebung in Abhängigkeit von auf zwei Brennflecken BF_{A}, BF_{B} eintreffenden Elektronen und eine nicht dargestellte Steuereinheit auf.

Die Elektronenemittervorrichtung 12 umfasst einen ersten Feldeffekt-Emitter 13 mit einer ersten Emitterfläche 13.A und einen zweiten Feldeffekt-Emitter 14 mit einer zweiten Emitterfläche 14.A. Die erste Emitterfläche 13.A und die zweite Emitterfläche 14.A weisen ein im Wesentlichen Röntgenstrahlen-transparentes Material auf. Die erste Emitterfläche 13.A und die zweite Emitterfläche 13.B sind derart segmentiert, dass ein Anteil der Elektronen-emittierenden Fläche 13.E, 14.E relativ zur jeweiligen gesamten Emitterfläche 13.A, 14.A durch selektives Schalten der Emittersegmente einstellbar ist. Die erste Emitterfläche 13.A und die zweite Emitterfläche 14.A sind im Strahlengang der in dem jeweiligen Brennfleck BF_{A}, BF_{B} generierten Röntgenstrahlung und somit zwischen der Anodeneinheit 15 und dem Patienten P angeordnet. Die Steuereinheit ist zum Einstellen eines Abstands A_{BF} zwischen den beiden Brennflecken BF_{A}, BF_{B} durch ein Vorgeben des Anteils der Elektronen-emittierenden Fläche 13.E, 14.E der ersten Emitterfläche 13.A und/oder der zweiten Emitterfläche 13.B ausgebildet ist. Die Propagationsrichtung der emittierten Elektronen e- ist mit Pfeilen gekennzeichnet.

In diesem Ausführungsbeispiel bilden die erste Emitterfläche 13.A und die zweite Emitterfläche 14.A ein vakuumdichtes Gehäuseteil des Röntgenröhrengehäuses 11. Außerdem ist in diesem Ausführungsbeispiel bildlich dargestellt, dass jeweils die gesamte Emitterfläche 13.A, 14.A die Elektronen-emittierenden Flächen 13.E, 14.E bilden. Der Abstand A_{BF} ist definiert als Abstand zwischen den geometrischen Mittelpunkten der beiden Brennflecken BF_{A}, BF_{B}.

In einer besonders vorteilhaften Weiterbildung ist das Röntgenstrahlen-transparente Material Silizium. Zusätzlich kann ein nicht gezeigter Fokuskopf des ersten Feldeffekt-Emitters 13 und/oder des zweiten Feldeffekt-Emitters 14 Aluminium aufweisen. Außerdem kann die Röntgenröhre 10 eine Schnittstelle zum Empfang eines Abstandsignals, welches mit dem Abstand A_{BF} zwischen den Brennflecken BF_{A}, BF_{B} korreliert, und/oder eine weitere Schnittstelle zum Empfang eines Blickbewegungssignals sowie eine weitere Steuereinheit zum derartigen nicht-achsensymmetrischen Einstellen des Anteils der Elektronen-emittierenden Fläche 13.E, 14.E in Abhängigkeit des Blickbewegungssignals aufweisen, dass zumindest einer der beiden Brennflecke BF_{A}, BF_{B} relativ zur rasterförmigen Segmentierung verdreht ist.

Die erste Variante der Röntgenröhre 10 ist dadurch gekennzeichnet, dass die Anodeneinheit 15 eine einzige drehbar gelagerte Scheibenanode 15.S aufweist, deren Oberfläche im Bereich der Brennflecke BF_{A}, BF_{B} im Wesentlichen einen Tellerwinkel von 0° aufweist, und dass die Scheibenanode 15.S somit zumindest abschnittsweise, insbesondere in diesem Ausführungsbeispiel komplett flach ist.

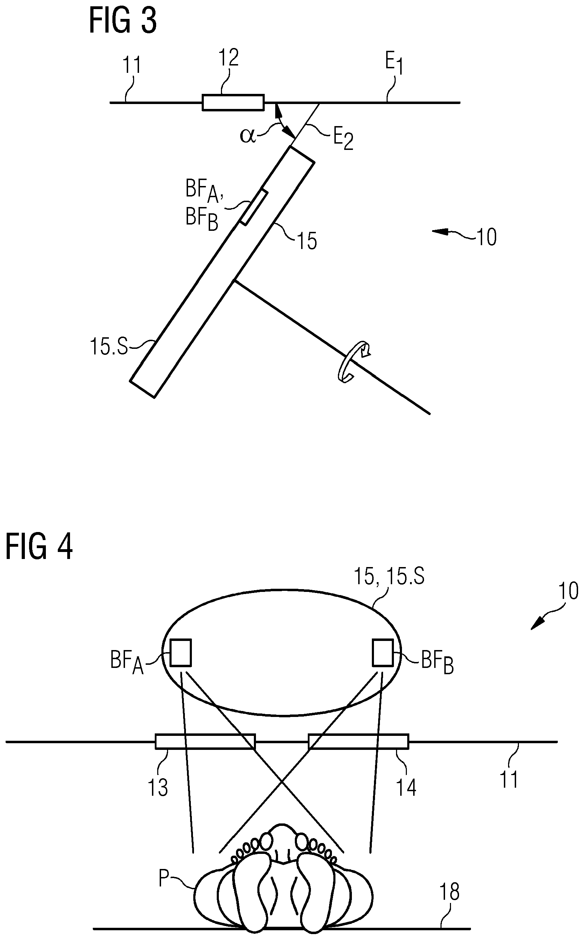

Insbesondere im Vergleich zur Ausführungsform in Fig. 3 ist darauf hingewiesen, dass eine erste Ebene E1, welche die erste Emitterfläche 13.A und die zweite Emitterfläche 14.A umfasst, sowie eine zweite Ebene E2, welche die flachen Abschnitte mit den Brennflecken BF_{A}, BF_{B} umfasst, sich geometrisch nicht schneiden.

**Fig. 2** zeigt die Röntgenröhre 10 der ersten Variante mit einer Draufsicht auf die Anodeneinheit 15 aus Sicht der Elektronenemittervorrichtung 12.

Die beiden Brennflecke BF_{A}, BF_{B} liegen innerhalb je einer maximalen Brennfleckfläche BF_{Amax}, BF_{Bmax}, welche insbesondere darstellen, in welchem Bereich der Oberfläche der Scheibenanode 15.S die Brennflecke BF_{A}, BF_{B} in Abhängigkeit von dem Anteil der Elektronen-emittierenden Flächen 13.E, 14.E liegen, insbesondere beliebig eingestellt werden, können. Im Vergleich zum in Fig. 1 gezeigten Ausführungsbeispiel ist für illustratorische Zwecke der Anteil der Elektronen-emittierenden Flächen 13.E, 14.E kleiner als die jeweilige Brennfleckfläche BF_{Amax}, BF_{Bmax}.

Diese Ausführungsform steht exemplarisch für eine nicht zusammenhängende Emitterfläche, weil zwischen den beiden maximalen Brennfleckflächen BF_{Amax}, BF_{Bmax} ein brennfleckfreier Bereich 16 vorgesehen ist. Eine maximale Ausdehnung 17 der Elektronen-emittierenden Fläche 13.E, 14.E, gemessen über beide Brennflecke BF_{A}, BF_{B} ist ebenso eingezeichnet.

**Fig. 3** zeigt die erste Variante der Röntgenröhre 10 gemäß den Figuren 1 bis 2 in einer vorteilhaften Weiterbildung.

Die Anodeneinheit 15 ist relativ zur ersten Emitterfläche 13.A und zur zweiten Emitterfläche 14.A derart gekippt angeordnet, dass sich eine erste Ebene E1, welche die erste Emitterfläche 13.A und die zweite Emitterfläche 14.A umfasst, sowie eine zweite Ebene E2, welche die flachen Abschnitte mit den Brennflecken BF_{A}, BF_{B} umfasst, sich geometrisch schneiden. Der Winkel, in denen sich die beiden Ebenen E1, E2 mathematisch betrachtet schneiden, ist mit α gekennzeichnet und größer 0°. Trotz der gekippten Anordnung ist ein jeweiliger Abstand zwischen den beiden Brennflecken BF_{A}, BF_{B} und der ersten Emitterfläche 13.A sowie der zweiten Emitterfläche 14.A im Wesentlichen gleich.

**Fig. 4** zeigt das Ausführungsbeispiel der Fig. 3 in einer weiteren schematischen Ansicht. In den beiden Brennflecken BF_{A}, BF_{B} wird die Röntgenstrahlung generiert, wobei die beiden Fächer den Strahlengang der Röntgenstrahlung, insbesondere den Nutzanteil davon, illustrieren sollen. Die Röntgenstrahlung durchdringt die im Strahlengang angeordneten Emitterflächen 13.A, 14.A, daraufhin den Patienten P und ist am Röntgendetektor 18 detektierbar.

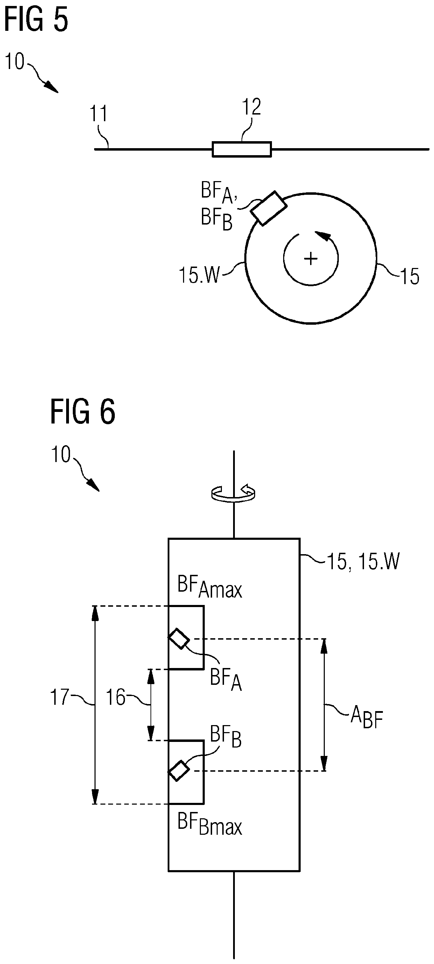

**Fig. 5** zeigt eine zweite Variante der Röntgenröhre 10 in einer Seitenansicht.

Im Vergleich zur ersten Variante der Röntgenröhre 10 weist die Anodeneinheit 15 eine einzige drehbar gelagerte Walzenanode 15.W mit einer zylindrischen Mantelfläche auf.

**Fig. 6** zeigt eine Draufsicht der zweiten Variante der Röntgenröhre 10 gemäß Fig. 5. Die Brennflecke BF_{A}, BF_{B} sind entlang der Mantelfläche axial versetzt angeordnet. In diesem Ausführungsbeispiel kann beispielhaft der Effekt nachvollzogen werden, wenn der Anteil der Elektronen-emittierenden Fläche 13.E, 14.E in Abhängigkeit des Blickbewegungssignals derart nicht-achsensymmetrisch eingestellt ist, dass beide Brennflecke BF_{A}, BF_{B}, insbesondere deren äußere Form, relativ zur rasterförmigen Segmentierung verdreht ist.

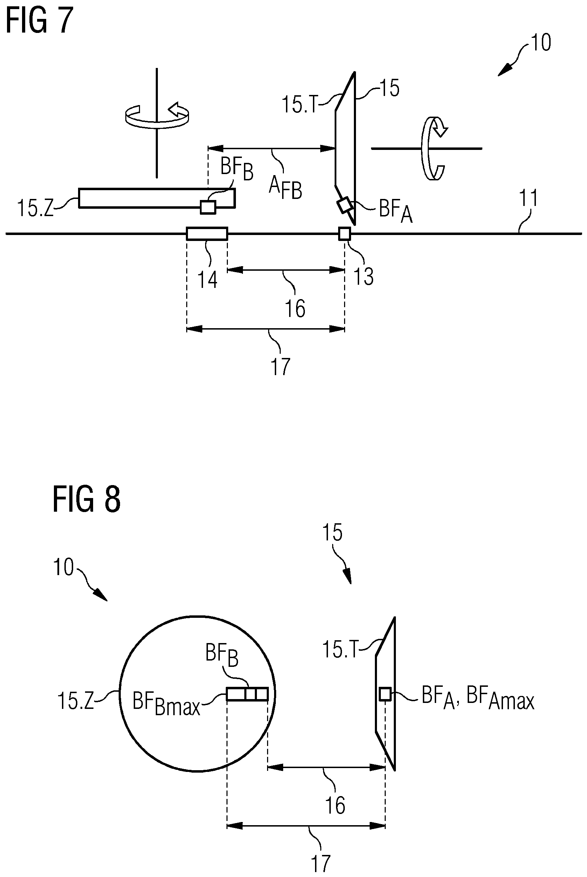

**Fig. 7** zeigt eine dritte Variante der Röntgenröhre 10 in einer Seitenansicht.

Im Vergleich zur ersten und zweiten Variante der Röntgenröhre 10 weist die Anodeneinheit 15 eine drehbar gelagerte Telleranode 15.T, deren Oberfläche im Bereich eines Brennflecks BF_{B} einen Tellerwinkel größer 0° aufweist, sowie eine weitere Anode 15.Z auf, wobei die beiden Brennflecke BF_{A}, BF_{B} auf die Telleranode 15.T und die weitere Anode 15.Z verteilt sind.

Die Telleranode 15.T ist derart ausgerichtet, dass deren Rotationsachse parallel zu einer Ebene ist, welche die erste Emitterfläche 13.A und die zweite Emitterfläche 14.A umfasst. Alternativ könnte die Rotationsachse bis zu einschließlich 90° verdreht sein, so dass diese Rotationsachse parallel zur Rotationsachse der drehbar gelagerten Scheibenanode ist.

In diesem Ausführungsbeispiel ist die weitere Anode 15.Z eine drehbar gelagerte Scheibenanode, deren Oberfläche im Bereich des einen Brennflecks BF_{B} im Wesentlichen einen Tellerwinkel von 0° aufweist, und somit zumindest abschnittsweise flach ist. Alternativ kann die weitere Anode 15.Z eine Stehanode sein.

**Fig. 8** zeigt eine Draufsicht der dritten Variante der Röntgenröhre 10.

Diese Figur zeigt, dass der Brennfleck BF_{A} der maximalen Brennfleckfläche BF_{Amax} insofern entspricht, weil die Position des Brennflecks BF_{A} in Richtung des Brennflecks BF_{B} im Wesentlichen unveränderlich ist. In dieser Ausführungsform ist die Intensität des Elektronenstroms, welche einen Einfluss auf die äußere Form des Brennflecks BF_{A} innerhalb der Grenzen der maximalen Brennfleckfläche BF_{Amax} haben kann, typischerweise dennoch variabel.

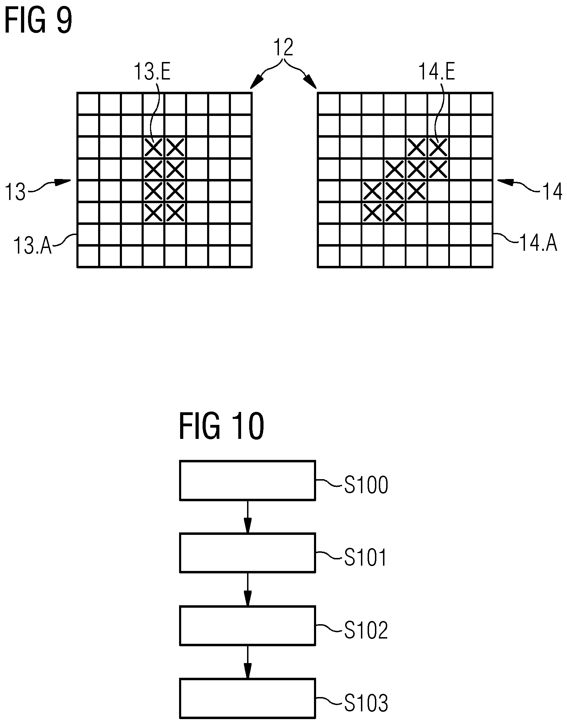

**Fig. 9** zeigt eine Elektronenemittervorrichtung 12 in einer Detailansicht, welche in jeder der drei zuvor gezeigten Varianten der Röntgenröhre vorgesehen sein kann. Die Elektronenemittervorrichtung 12 weist den ersten Feldeffekt-Emitter 13 und den zweiten Feldeffekt-Emitter 14 auf, welche keine zusammenhängende Emitterfläche aufweisen. Die beiden Feldeffekt-Emitter 13, 14 sind also physisch getrennt.

Die erste Emitterfläche 13.A und die zweite Emitterfläche 14.A sind jeweils rasterförmig in einer 8x8 Matrix segmentiert. Grundsätzlich ist es denkbar, dass sich die Anzahl der Zeilen von der Anzahl der Spalten unterscheidet. Die Anzahl der Zeilen bzw. der Spalten kann beispielsweise mehr als 8, insbesondere 256, 512, 1024 oder mehr betragen.

Das Ausführungsbeispiel in Fig. 9 zeigt jeweils den Anteil der Elektronen-emittierenden Fläche 13.E, 14.E, indem die jeweiligen Elektronen-emittierenden und somit angeschalteten Segmente mit einem X gekennzeichnet sind. Die nicht mit X gekennzeichneten abgeschalteten Segmente emittieren keine Elektronen.

Der Anteil der Elektronen-emittierenden Fläche 14.E ist, beispielsweise in Abhängigkeit des Blickbewegungssignals, derart nicht-achsensymmetrisch eingestellt, dass der daraus resultierende Brennfleck BF_{A}, BF_{B} relativ zur rasterförmigen Segmentierung verdreht ist.

**Fig. 10** zeigt ein Verfahren zur Generierung von Röntgenstrahlung für eine stereoskopische Bildgebung mittels einer Röntgenröhre 10 in einem Flussdiagramm.

Verfahrensschritt S100 kennzeichnet ein Bereitstellen eines Augenabstands eines Nutzers der Röntgenröhre 10 in Form des Abstandsignals. Vor dem Bereitstellen kann der Augenabstand beispielsweise mittels einer Kamera ermittelt werden.

Verfahrensschritt S101 kennzeichnet ein Übermitteln des Abstandsignals an die Schnittstelle der Röntgenröhre 10.

Verfahrensschritt S102 kennzeichnet ein Einstellen des Abstands zwischen den beiden Brennflecken, insbesondere in der Steuereinheit der Röntgenröhre 10.

Verfahrensschritt S103 kennzeichnet ein Konsekutives generieren der Röntgenstrahlung für die stereoskopische Bildgebung in den beiden beabstandeten Brennflecken.

Obwohl die Erfindung im Detail durch die bevorzugten Ausführungsbeispiele näher illustriert und beschrieben wurde, so ist die Erfindung dennoch nicht durch die offenbarten Beispiele eingeschränkt und andere Variationen können vom Fachmann hieraus abgeleitet werden, ohne den Schutzumfang der Erfindung zu verlassen.

## Patentansprüche

1. Röntgenröhre (10) für eine stereoskopische Bildgebung, aufweisend
- ein evakuiertes Röntgenröhrengehäuse (11),
- eine in dem Röntgenröhrengehäuse (11) angeordnete Elektronenemittervorrichtung (12),
- eine in dem Röntgenröhrengehäuse (11) angeordnete Anodeneinheit (15) zur Generierung von Röntgenstrahlung für die stereoskopische Bildgebung in Abhängigkeit von auf zwei Brennflecken (BF_{A}, BF_{B}) eintreffenden Elektronen und
- eine Steuereinheit,
- wobei die Elektronenemittervorrichtung (12) einen ersten Feldeffekt-Emitter (13) mit einer ersten Emitterfläche (13.A) und einen zweiten Feldeffekt-Emitter (14) mit einer zweiten Emitterfläche (14.A) umfasst,
- wobei die erste Emitterfläche (13.A) und die zweite Emitterfläche (14.A) ein im Wesentlichen Röntgenstrahlen-transparentes Material aufweisen,
- wobei die erste Emitterfläche (13.A) und/oder die zweite Emitterfläche (14.A) derart segmentiert ist, dass ein Anteil der Elektronen-emittierenden Fläche (13.E, 14.E) relativ zur jeweiligen gesamten Emitterfläche (13.A, 14.A) durch selektives Schalten der Emittersegmente einstellbar ist,
**dadurch gekennzeichnet,**
- **dass** die erste Emitterfläche (13.A) und die zweite Emitterfläche (14.A) im Strahlengang der in dem jeweiligen Brennfleck (BF_{A}, BF_{B}) generierten Röntgenstrahlung angeordnet sind sowie
- **dass** die Steuereinheit zum Einstellen eines Abstands (A_{BF}) zwischen den beiden Brennflecken (BF_{A}, BF_{B}) durch ein Vorgeben des Anteils der Elektronen-emittierenden Fläche der ersten Emitterfläche und/oder der zweiten Emitterfläche ausgebildet ist.

2. Röntgenröhre (10) nach Anspruch 1, wobei das Röntgenstrahlen-transparente Material Silizium ist.

3. Röntgenröhre (10) nach einem der vorhergehenden Ansprüche, wobei die erste Emitterfläche (13.A) und/oder die zweite Emitterfläche (14.A) ein vakuumdichtes Gehäuseteil des Röntgenröhrengehäuses (11) bilden.

4. Röntgenröhre (10) nach einem der vorhergehenden Ansprüche, wobei der erste und/oder der zweite Feldeffekt-Emitter einen Fokuskopf aufweisen und der Fokuskopf des ersten Feldeffekt-Emitters (13) und/oder des zweiten Feldeffekt-Emitters (14) Aluminium aufweist.

5. Röntgenröhre (10) nach einem der vorhergehenden Ansprüche, ferner aufweisend eine Schnittstelle zum Empfang eines Abstandsignals, welches mit dem Abstand (A_{BF}) zwischen den Brennflecken (BF_{A}, BF_{B}) korreliert.

6. Röntgenröhre (10) nach einem der vorhergehenden Ansprüche, wobei die erste Emitterfläche (13.A) und die zweite Emitterfläche (14.A) rasterförmig segmentiert sind, wobei die Röntgenröhre aufweist eine Schnittstelle, oder eine weitere Schnittstelle, wenn Anspruch 6 von Anspruch 5 abhängt, zum Empfang eines Blickbewegungssignals und eine Steuereinheit, oder eine weitere Steuereunheit, wenn Anspruch 6 von Anspruch 5 abhängt, zum derartigen nicht-achsensymmetrischen Einstellen des Anteils der Elektronen-emittierenden Fläche (13.E, 14.E) in Abhängigkeit des Blickbewegungssignals, dass zumindest einer der beiden Brennflecke (BF_{A}, BF_{B}) relativ zur rasterförmigen Segmentierung verdreht ist.

7. Röntgenröhre (10) nach einem der vorhergehenden Ansprüche, wobei die Anodeneinheit (15) eine einzige drehbar gelagerte Scheibenanode (15.S) aufweist, deren Oberfläche im Bereich der Brennflecke (BF_{A}, BF_{B}) im Wesentlichen einen Tellerwinkel von 0° aufweist, und wobei die Scheibenanode (15.S) somit zumindest abschnittsweise flach ist.

8. Röntgenröhre (10) nach Anspruch 7, wobei die Anodeneinheit (15) relativ zur ersten Emitterfläche (13.A) und zur zweiten Emitterfläche (14.A) derart gekippt angeordnet ist, dass sich eine erste Ebene (E1), welche die erste Emitterfläche (13.A) und die zweite Emitterfläche (14.A) umfasst, sowie eine zweite Ebene (E2), welche die flachen Abschnitte mit den Brennflecken (BF_{A}, BF_{B}) umfasst, sich geometrisch schneiden.

9. Röntgenröhre (10) nach einem der Ansprüche 1 bis 6, wobei die Anodeneinheit (15) eine einzige drehbar gelagerte Walzenanode (15.W) mit einer zylindrischen Mantelfläche aufweist und wobei die Brennflecke (BF_{A}, BF_{B}) entlang der Mantelfläche axial versetzt angeordnet sind.

10. Röntgenröhre (10) nach einem der Ansprüche 1 bis 6, wobei die Anodeneinheit (15) eine drehbar gelagerte Telleranode (15.T), deren Oberfläche im Bereich eines Brennflecks (BF_{A}) einen Tellerwinkel größer 0° aufweist, sowie eine weitere Anode (15.Z) aufweist, wobei die beiden Brennflecke (BF_{A}, BF_{B}) auf die Telleranode (15.T) und die weitere Anode (15.Z) verteilt sind.

11. Röntgenröhre (10) nach Anspruch 10, wobei die Telleranode (15.T) derart ausgerichtet ist, dass deren Rotationsachse parallel zu einer Ebene ist, welche die erste Emitterfläche (13.A) und die zweite Emitterfläche (14.A) umfasst.

12. Röntgenröhre (10) nach einem der Ansprüche 10 oder 11, wobei die weitere Anode (15.Z) eine Stehanode oder eine drehbar gelagerte Scheibenanode ist, deren Oberfläche im Bereich des einen Brennflecks (BF_{B}) im Wesentlichen einen Tellerwinkel von 0° aufweist, und somit zumindest abschnittsweise flach ist.

13. Verfahren zur Generierung von Röntgenstrahlung für eine stereoskopische Bildgebung mittels einer Röntgenröhre (10) nach Anspruch 5, mit den folgenden Schritten:
- Bereitstellen eines Augenabstands eines Nutzers (N) der Röntgenröhre (10) in Form des Abstandsignals,
- Übermitteln des Abstandsignals an die Schnittstelle der Röntgenröhre (10),
- Einstellen des Abstands (A_{BF}) zwischen den beiden Brennflecken (BF_{A}, BF_{B}) und
- Konsekutives generieren der Röntgenstrahlung für die stereoskopische Bildgebung in den beiden beabstandeten Brennflecken (BF_{A}, BF_{B}).

14. Verfahren nach Anspruch 13, wobei der Augenabstand mittels einer Kamera ermittelt wird.

## Claims

1. X-ray tube (10) for a stereoscopic imaging, having
- an evacuated x-ray tube housing (11),
- an electron emitter apparatus (12) arranged in the x-ray tube housing (11),
- an anode unit (15) arranged in the x-ray tube housing (11) for generating x-ray radiation for the stereoscopic imaging as a function of electrons striking two focal points (BF_{A}, BF_{B}) and
- a control unit,
- wherein the electron emitter apparatus (12) comprises a first field effect emitter (13) with a first emitter surface (13.A) and a second field effect emitter (14) with a second emitter surface (14.A),
- wherein the first emitter surface (13.A) and the second emitter surface (14.A) have a substantially x-ray-transparent material,
- wherein the first emitter surface (13.A) and/or the second emitter surface (14A) is segmented so that a portion of the electron-emitting surface (13.E, 14.E) can be set relative to the respective overall emitter surface (13.A, 14.A) by selectively switching the emitter segments,
**characterised in that**
- the first emitter surface (13.A) and the second emitter surface (14.A) are arranged in the beam path of the x-ray radiation generated in the respective focal point (BF_{A}, BF_{B}) and
- the control unit is embodied to set a distance (A_{BF}) between the two focal points (BF_{A}, BF_{B}) by indicating the portion of the electron-emitting surface of the first emitter surface and/or the second emitter surface.

2. X-ray tube (10) according to claim 1, wherein the x-ray-transparent material is silicon.

3. X-ray tube (10) according to one of the preceding claims, wherein the first emitter surface (13.A) and/or the second emitter surface (14.A) form a vacuum-tight housing part of the x-ray tube housing (11).

4. X-ray tube (10) according to one of the preceding claims, wherein the first and/or the second field effect emitter have a focal head and the focal head of the first field effect emitter (13) and/or the second field effect emitter (14) has aluminium.

5. X-ray tube (10) according to one of the preceding claims, further having an interface for receiving a distance signal which correlates with the distance (A_{BF}) between the focal points (BF_{A}, BF_{B}).

6. X-ray tube (10) according to one of the preceding claims, wherein the first emitter surface (13.A) and the second emitter surface (14.A) are segmented in the shape of a grid, wherein the x-ray tube has an interface or a further interface, if claim 6 depends on claim 5, for receiving an eye movement signal and a control unit or a further control unit, if claim 6 depends on claim 5, for the non-axially symmetrical setting of the portion of the electron-emitting surface (13.E, 14.E) as a function of the eye movement signal so that at least one of the two focal points (BF_{A}, BF_{B}) is twisted relative to the grid-shaped segmentation.

7. X-ray tube (10) according to one of the preceding claims, wherein the anode unit (15) has a single rotatably mounted disc anode (15.S), the surface of which in the region of the focal point (BF_{A}, BF_{B}) has substantially a disc angle of 0°, and wherein the disc anode (15.S) is therefore flat at least in sections.

8. X-ray tube (10) according to claim 7, wherein the anode unit (15) is arranged tilted relative to the first emitter surface (13.A) and the second emitter surface (14.A) so that a first plane (E1) which comprises the first emitter surface (13.A) and the second emitter surface (14.A) and a second plane (E2), which comprises the flat sections with the focal points (BF_{A}, BF_{B}), intersect one another geometrically.

9. X-ray tube (10) according to one of claims 1 to 6, wherein the anode unit (15) has a single rotatably mounted roller anode (15.W) with a cylindrical peripheral surface and wherein the focal points (BF_{A}, BF_{B}) are arranged axially offset along the peripheral surface.

10. X-ray tube (10) according to one of claims 1 to 6, wherein the anode unit (15) has a rotatably mounted disc anode (15.T), the surface of which in the region of a focal point (BF_{A}) has a disc angle of greater than 0°, and a further anode (15.Z), wherein the two focal points (BF_{A}, BF_{B}) are distributed on the disc anode (15.T) and the further anode (15.Z).

11. X-ray tube (10) according to claim 10, wherein the disc anode (15.T) is aligned so that its axis of rotation is parallel to a plane which comprises the first emitter surface (13.A) and the second emitter surface (14.A).

12. X-ray tube (10) according to one of claims 10 or 11, wherein the further anode (15.Z) is a stationary anode or a rotatably mounted disc anode, the surface of which in the region of the one focal point (BF_{B}) has substantially a disc angle of 0° and is thus flat at least in sections.

13. Method for generating x-ray radiation for a stereoscopic imaging by means of an x-ray tube (10) as claimed in claim 5, with the following steps:
- providing an eye distance of a user (N) of the x-ray tube (10) in the form of the distance signal,
- transmitting the distance signal to the interface of the x-ray tube (10),
- setting the distance (A_{BF}) between the two focal points (BF_{A}, BF_{B}) and
- consecutively generating the x-ray radiation for the stereoscopic imaging in the two distanced focal points (BF_{A}, BF_{B}).

14. Method according to claim 13, wherein the eye distance is determined by means of a camera.

## Revendications

1. Tube (10) à rayons X pour une imagerie stéréoscopique, comportant
- un boîtier (11) de tube à rayons X sous vide,
- un dispositif (12) émetteur d'électrons disposé dans le boîtier (11) du tube à rayons X,
- une unité (15) d'anode disposée dans le boîtier (11) du tube à rayons X pour la production de rayonnement X pour l'imagerie stéréoscopique en fonction d'électrons incidents sur deux taches (BF_{A}, BF_{B}) focales et
- une unité de commande,
- dans lequel le dispositif (12) émetteur d'électrons comprend un premier émetteur (13) à effet de champ ayant une première surface (13.A) d'émetteur et un deuxième émetteur (14) à effet de champ ayant une deuxième surface (14.A) d'émetteur,
- dans lequel la première surface (13.A) d'émetteur et la deuxième surface (14.A) d'émetteur ont un matériau sensiblement transparent au rayonnement X,
- dans lequel la première surface (13.A) d'émetteur et/ou la deuxième surface (14.A) d'émetteur sont segmentées, de manière à pouvoir par commutation sélective des segments d'émetteur, régler une proportion de la surface (13.E, 14.E) émettant des électrons par rapport à toute la surface (13.A, 14.A) d'émetteur respective,
**caractérisé**
- **en ce que** la première surface (13.A) d'émetteur et la deuxième surface (14.A) d'émetteur sont montées dans le chemin du rayonnement X produit à la tache (BF_{A}, BF_{B}) focale respective, ainsi que
- **en ce que** l'unité de commande est constituée pour régler une distance (A_{BF}) entre les deux taches (BF_{A}, BF_{B}) focales par la prescription de la proportion de la surface émettant des électrons de la première surface d'émetteur et/ou de la deuxième surface d'émetteur.

2. Tube (10) à rayons X suivant la revendication 1, dans lequel le matériau transparent au rayon X est le silicium.

3. Tube (10) à rayons X suivant l'une des revendications précédentes, dans lequel la première surface (13.A) d'émetteur et/ou la deuxième surface (14.A) d'émetteur forment une partie étanche au vide du boîtier (11) du tube à rayons X.

4. Tube (10) à rayons X suivant l'une des revendications précédentes, dans lequel le premier et/ou le deuxième émetteur à effet de champ ont une tête de foyer et la tête de foyer du premier émetteur (13) à effet de champ et/ou du deuxième émetteur (14) à effet de champ comporte de l'aluminium.

5. Tube (10) à rayons X suivant l'une des revendications précédentes, comportant une interface de réception d'un signal de distance, qui est corrélée à la distance (A_{BF}) entre les taches (BF_{A}, BF_{B}) focales.

6. Tube (10) à rayons X suivant l'une des revendications précédentes, dans lequel la première surface (13.A) d'émetteur et la deuxième surface (14.A) d'émetteur sont segmentées en forme de trame, dans lequel le tube à rayons X a une interface ou une autre interface, lorsque la revendication 6 dépend de la revendication 5, de réception d'un signal de déplacement visuel et une unité de commande, ou une autre unité de commande, lorsque la revendication 6 dépend de la revendication 5, pour le réglage d'une manière non symétrique à l'axe de la proportion de la surface (13.E, 14.E) émettant des électrons en fonction du signal de déplacement visuel, de manière à ce qu'au moins l'une des deux taches (BF_{A}, BF_{B}) focales soit tournée par rapport à la segmentation en forme de trame.

7. Tube (10) à rayons X suivant l'une des revendications précédentes, dans lequel l'unité (15) d'anode a une anode (15.S) à disque unique montée tournante, dont la surface a dans la partie de la tache (BF_{A}, BF_{B}) focale sensiblement un angle d'assiette de 0°, et dans lequel l'anode (15.S) coulissante est ainsi plate au moins par endroit.

8. Tube (10) à rayons X suivant la revendication 7, dans lequel l'unité (15) d'anode est montée basculée par rapport à la première surface (13.A) d'émetteur et par rapport à la deuxième surface (14.A) d'émetteur, de manière à ce qu'un premier plan (E1), qui comprend la première surface (13.A) d'émetteur et la deuxième surface (14.A) d'émetteur, ainsi qu'un deuxième plan (E2), qui comprend les parties plates ayant les taches (BF_{A}, BF_{B}) focales, se coupent géométriquement.

9. Tube (10) à rayons X suivant l'une des revendications 1 à 6, dans lequel l'unité (15) d'anode a une seule anode (15.W) en rouleau montée tournante ayant une surface latérale cylindrique et dans lequel les taches (BF_{A}, BF_{B}) focales sont disposées de manière décalée axialement le long de la surface latérale.

10. Tube (10) à rayons X suivant l'une des revendications 1 à 6, dans lequel l'unité (15) d'anode a une anode (15.T) en assiette montée tournante, dont la surface supérieure a, dans la partie d'une tache (BF_{A}) focale, un angle d'assiette plus grand que 0°, ainsi qu'une autre anode (15.Z), dans lequel les deux taches (BF_{A}, BF_{B}) focales sont réparties sur l'anode (15.T) en assiette et l'autre anode (15.Z).

11. Tube (10) à rayons X suivant la revendication 10, dans lequel l'anode (15.T) en assiette est orientée, de manière à ce que son axe de révolution soit parallèle à un plan, qui comprend la première surface (13.A) d'émetteur et la deuxième surface (14.A) d'émetteur.

12. Tube (10) à rayons X suivant l'une des revendications 10 ou 11, dans lequel l'autre anode (15.Z) est une anode fixe ou une anode coulissante montée tournante, dont la surface a dans la partie de la une tache (BF_{B}) focale, sensiblement un angle d'assiette de 0° et est ainsi plate au moins par endroit.

13. Procédé de production du rayonnement X pour une imagerie stéréoscopique au moyen d'un tube (10) à rayons X suivant la revendication 5, comprenant les stades suivants :
- disposer d'une distance oculaire d'un utilisateur (N) du tube (10) à rayons X sous la forme du signal de distance,
- transmettre le signal de distance à l'interface du tube (10) à rayons X,
- régler la distance (A_{BF}) entre les deux taches (BF_{A}, BF_{B}) focales et
- produire consécutivement le rayonnement X pour l'imagerie stéréoscopique dans les deux taches (BF_{A}, BF_{B}) focales à distance.

14. Procédé suivant la revendication 13, dans lequel on détermine la distance oculaire au moyen d'un appareil photographique.
